# EUROPÄISCHE PATENTSCHRIFT

(11) **EP 3 074 557 B1**
(45) Veröffentlichungstag und Bekanntmachung des Hinweises auf die Patenterteilung: **15.02.2023**
(21) Anmeldenummer: 14827160.4
(22) Anmeldetag: 26.11.2014
(51) Int. Cl.: D03D 15/00, C12M 1/00, D03D 15/292

(54) **PHOTOBIOREAKTOR MIT MATTEN AUS LICHT-AUSKOPPELNDEN LICHTLEITERFASERN UND EIN ELEKTRISCHES WANDERFELD ERZEUGENDEN ELEKTRISCH LEITFÄHIGEN FASERN**
PHOTOBIOREACTOR WITH MATS OF LIGHT-OUTCOUPLING OPTICAL FIBERS, AND ELECTRICALLY CONDUCTIVE FIBERS WHICH GENERATE AN ELECTRIC TRAVELING FIELD
PHOTOBIORÉACTEUR COMPORTANT DES NATTES DE FIBRES PHOTOCONDUCTRICES À DÉCOUPLAGE DE LUMIÈRE ET FIBRES ÉLECTRIQUEMENT CONDUCTRICES GÉNÉRANT UN CHAMP ÉLECTRIQUE ALTERNATIF

(30) Priorität: 28.11.2013 DE 102013019889
(43) Veröffentlichungstag der Anmeldung: 05.10.2016
(73) Patentinhaber: Airbus Defence and Space GmbH, 85521 Ottobrunn (DE)
(72) Erfinder: GÖBEL, Johann, 81547 München (DE); WAGNER, Jennifer, 20257 Hamburg (DE); SCHREIBER, Robert, 82166 Gräfelfing (DE)
(74) Vertreter: LKGLOBAL Lorenz & Kopf PartG mbB Patentanwälte
(86) Internationale Anmeldenummer: PCT/DE2014/100416
(87) Internationale Veröffentlichungsnummer: WO 2015/078451

(56) Entgegenhaltungen:
- DE-A1-102010 025 366
- US-A1- 2006 144 460
- US-A1- 2011 074 380
- DATABASE WPI Week 201230 Thomson Scientific, London, GB; AN 2012-D89017 XP002737385, & CN 102 382 754 A (CHINESE ACAD SCI PROCESS ENG INST) 21. März 2012 (2012-03-21)

## Beschreibung

### VERWANDTE ANMELDUNGEN

Die vorliegende Anmeldung beansprucht die Priorität der deutschen

Patentanmeldung Nr. 10 2013 019 889.5, eingereicht am 28. November 2013.

### GEBIET DER ERFINDUNG

Die vorliegende Erfindung betrifft einen Photobioreaktor zur Kultivierung von phototrophen Organismen. Ferner betrifft die Erfindung Matten aus Fasern sowie eine Mattenvorrichtung mit solchen Matten, wie sie vorteilhaft in einem Photobioreaktor eingesetzt werden können.

### HINTERGRUND DER ERFINDUNG

Phototrophe Organismen sind Kleinstlebewesen, z.B. in Form von Mikroorganismen, die Licht als Energiequelle für ihren Stoffwechsel direkt nutzen können. Zu den phototrophen Organismen zählen zum Beispiel bestimmte Pflanzen, Moose, Mikroalgen, Makroalgen, Cyanobakterien und Purpurbakterien.

Für unterschiedliche Anwendungszwecke kann es gewünscht sein, Biomasse beispielsweise in Form von Algen in großen Mengen und preisgünstig herstellen zu können. Beispielsweise kann solche Biomasse für die Erzeugung alternativer Biotreibstoffe z.B. für den Transportsektor verwendet werden.

Um Biomasse im industriellen Maßstab erzeugen zu können, werden so genannte Bioreaktoren eingesetzt. Ein Bioreaktor ist eine Anlage zur Produktion von

Organismen außerhalb ihrer natürlichen und innerhalb einer künstlichen technischen Umgebung. So genannte Photobioreaktoren werden eingesetzt, um phototrophe Organismen zu kultivieren. Ein Photobioreaktor stellt den phototrophen Organismen dabei sowohl Licht als auch CO₂ sowie gegebenenfalls eine geeignete Nährlösung zur Verfügung, damit diese entsprechend Biomasse aufbauen können.

Generell sind für Photobioreaktoren sowohl offene als auch geschlossene Systeme bekannt. Jeder dieser Typen von Photobioreaktoren weist bestimmte Vorteile und Nachteile auf.

Bei offenen Photobioreaktorsystemen, teilweise auch als open ponds bezeichnet, werden phototrophe Organismen in offenen Becken oder Teichen kontrolliert gezüchtet. Dabei wird meist eine Nährlösung oder Kultursuspension, die alle für den jeweiligen Organismus notwendigen Nährstoffe und CO₂ enthält, in einem Kreislauf gefördert und von der offenen Oberfläche her meist direkt von der Sonne beleuchtet.

Mögliche Vorteile solcher offenen Photobioreaktorsysteme sind ein verhältnismäßig geringer technischer Aufwand sowie ein geringer Stromverbrauch.

Allerdings bringt eine Beleuchtung lediglich über die nach oben offene Fläche mit sich, dass nur geringe Volumina mit ausreichend Licht versorgt werden können. Licht kann in eine mit Organismen versetzte Nährlösung meist nur wenige Zentimeter tief eindringen. Die Tiefe solcher offenen Photobioreaktorsysteme ist somit in der Regel auf 20 bis 30 cm begrenzt. Der geringe mittlere Lichteintrag führt zu geringen flächenbezogenen Wachstumsraten. Für offene Photobioreaktorsysteme muss somit viel Fläche bereitgestellt werden. Hierdurch werden Kosten für solche Photobioreaktoren insbesondere in dicht besiedelten Regionen erheblich erhöht.

Außerdem kann es an der frei liegenden Oberfläche zu einer starken Verdunstung und damit zu Aufsalzungseffekten kommen. Über die frei liegende Oberfläche kann ferner eine erhebliche Menge an CO₂ in die Atmosphäre diffundieren. Im Gegenzug können über die frei liegende Oberfläche Verschmutzungen in einen offenen Photobioreaktor gelangen, diesen kontaminieren und damit eine Produktreinheit gefährden. Ferner gestaltet sich eine eventuell notwendige Heizung oder Kühlung solcher offenen Photobioreaktorsysteme schwierig. Bei ausschließlicher Beleuchtung mit Sonnenlicht ergibt sich außerdem eine Tageszeitenabhängigkeit, wobei tiefer liegende Schichten häufig nur unzureichend beleuchtet werden, wohingegen direkt an der Oberfläche des offenen Systems sehr hohe Beleuchtungsintensitäten auftreten können, die gegebenenfalls zur so genannten Photoinhibition führen können.

Die Summe der genannten Nachteile beziehungsweise beschränkenden Randbedingungen kann insbesondere dazu führen, dass offene Photobioreaktorsysteme in Form von open ponds häufig nur in ganz bestimmten geographischen Bereichen ganzjährig eingesetzt werden können.

Um einerseits einen Einfluss von Umweltbedingungen zu reduzieren und um andererseits einen höheren Ertrag bei der Kultivierung von phototrophen Organismen zu erreichen, wurden geschlossene Photobioreaktorsysteme entwickelt. In solchen geschlossenen Systemen wird eine Nährlösung zusammen mit den Organismen durch einen geschlossenen Kreislauf geleitet und dabei meist von außen her beleuchtet.

Beispielsweise werden bei einem Rohr-Photobioreaktor Glas- oder Kunststoffrohre zu einem geschlossenen Kreislauf zusammengesetzt und die darin eingeschlossenen Organismen mittels einer zentralen Einheit, die beispielsweise geeignete Pumpen und Sensoren beinhalten kann, mit Nährstoffen und CO₂ versorgt.

Geschlossene Photobioreaktoren erlauben in der Regel eine hohe Prozesskontrolle, da die Organismen und die umgebende Nährlösung in dem geschlossenen System gut geheizt beziehungsweise gekühlt werden können, ein pH-Wert überwacht und gegebenenfalls angepasst werden kann und zusätzliches Licht zur Verfügung gestellt werden kann. Die geschlossenen Systeme erlauben bei geringem Flächenbedarf eine hohe Produktivität, da beispielsweise mehrere geschlossene Systeme übereinander angeordnet werden können oder Rohre eines Systems in vertikaler Richtung verlaufen können und dabei von allen Seiten her beleuchtet werden können. Dabei ist aber immer mit Abschattungseffekten zu rechnen. Außerdem sind auch eine hohe Produktreinheit bei geringen Kontaminationen, geringe Verdunstung sowie geringe elektromagnetische Beeinträchtigungen (EMV) möglich.

Allerdings sind ein technischer Aufwand und entsprechende Anlagen-Investitionskosten beim Aufbau komplexer geschlossener Photobioreaktoren im Vergleich zu offenen Systemen in der Regel sehr hoch.

Es wurde bereits eine Vielzahl von technischen Lösungen entwickelt, um eine Effizienz von Photobioreaktoren zu steigern. Als Maß für die Effizienz eines Photobioreaktors kann hierbei die Menge notwendiger Ressourcen wie beispielsweise bereitzustellende Energie in Form von Licht und/oder Elektrizität, bereitzustellende Fläche, bereitzustellende Nährstoffe, etc. in Relation zum Ertrag des Photobioreaktors in Form von Biomasse mit möglichst hohen Mengen darin chemisch gespeicherter Energie verstanden werden.

Beispielsweise wurde in der EP 2 520 642 A1 ein Photobioreaktor mit rotatorisch oszillierenden Lichtquellen beschrieben.

US 2006/0144460 beschreibt ein Verfahren zum Weben eines Textils, das zumindest teilweise Glasfasern wie beispielsweise Schussfäden und/oder Kettfäden enthält, die derart behandelt sind, dass sie Licht senkrecht zu einer Longitudinalachse davon streuen können.

### ZUSAMMENFASSUNG DER ERFINDUNG

Es kann als eine Aufgabe der vorliegenden Erfindung angesehen werden, einen Photobioreaktor zur Kultivierung von phototrophen Organismen bereitzustellen, der eine hohe Effizienz bei geringen Anlagen-Investitionskosten und / oder geringen Betriebskosten ermöglicht. Insbesondere kann erwünscht sein, phototrophe Organismen innerhalb eines Photobioreaktors sowohl effizient mit Licht versorgen zu können als auch die phototrophen Organismen bzw. eine diese aufnehmende Nährlösung gezielt bewegen zu können, um beispielsweise für eine Durchmischung zu sorgen oder die phototrophen Organismen gezielt aus dem Photobioreaktor entnehmen, d.h. "ernten", zu können. Ferner kann es erwünscht sein, Komponenten für einen Bioreaktor bereitzustellen, die diese Vorteile ermöglichen.

Diese Aufgaben können erfüllt werden durch einen Photobioreaktor gemäß Anspruch 11, eine Matte gemäß Anspruch 1 bzw. eine Mattenanordnung gemäß Anspruch 9. Vorteilhafte Ausführungsformen sind in den abhängigen Ansprüchen sowie in der nachfolgenden Beschreibung angegeben.

Ideen zu Ausführungsformen des erfindungsgemäßen Photobioreaktors können unter anderem als auf den folgenden Gedanken und Erkenntnissen beruhend angesehen werden:
Phototrophe Organismen sollen zu ihrer Aufzucht möglichst gut mit Licht und Nährstoffen versorgt werden. Allerdings kann sich Licht insbesondere in einer stark mit Organismen versetzten Nährlösung nur über sehr kurze Distanzen von wenigen Zentimetern ausbreiten. Ein Photobioreaktor, bei dem die Nährlösung in einem Behälter aufgenommen ist und der Behälter lediglich von außen her beleuchtet wird, muss daher bei verhältnismäßig kleinem Volumen eine möglichst große beleuchtbare Außenoberfläche bereitstellen. Damit einher geht die Notwendigkeit einer großen für den Photobioreaktor zur Verfügung zu stellenden Grundfläche, beispielsweise wie bei einem open-pond-System, oder eines komplexen strukturellen Aufbaus, wie bei herkömmlichen geschlossenen Systemen wie zum Beispiel Rohr-Photobioreaktoren.

Entsprechend Ausführungsformen der Erfindung wird nun vorgeschlagen, in einem die Nährlösung aufnehmenden Behälter eines Photobioreaktors eine oder mehrere spezielle Matten anzuordnen. Die Matte enthält dabei Fasern, die speziell dazu ausgebildet sind, an ihren Enden eingekoppeltes Licht nicht nur an gegenüber liegenden Enden der Fasern auszukoppeln sondern seitlich, das heißt quer zu einer Oberfläche der Lichtleitermatte auszukoppeln. Die Lichtauskopplung kann dabei möglichst homogen über eine gesamte Oberfläche der Lichtleitermatte hin erfolgen. Damit kann erreicht werden, dass große Mengen Licht im Inneren des Behälters des Photobioreaktors weitgehend homogen verteilt über die Oberfläche der lichtleitenden Matte eingebracht werden können. Durch die Verwendung der wenigstens einen seitlich licht-auskoppelnden Matte können für den vorgeschlagenen Photobioreaktor somit eine erhöhte Effizienz sowie möglicherweise weitere, weiter unten näher zu beschreibende Vorteile erreicht werden.

Die Matte soll ferner auch Fasern aufweisen, die elektrisch leitfähig sind. Indem an diese Fasern gesteuert elektrische Spannungen angelegt werden, können entlang einer Oberfläche der Matte gezielt elektrische Felder bewirkt werden. Solche elektrischen Felder können dazu ausgenutzt werden, um elektrisch geladene Teilchen oder Komponenten gezielt zu bewegen oder in eine Richtung zu fördern.

Beispielsweise liegen die phototrophen Organismen selbst in einer beleuchteten Lösung meist in einem elektrisch geladenen Zustand vor, so dass diese unter einer Einwirkung eines elektrischen Feldes bewegt werden können. Auf diese Weise können die phototrophen Organismen beispielsweise in dem Photobioreaktor kontinuierlich durchmischt werden und/oder hin zu einem Bereich des Photobioreaktors gefördert werden, aus dem sie dann entnommen, das heißt "geerntet", werden können.

Eine aus verschiedenen Fasern bestehende Matte kann somit in einem Photobioreaktor sowohl dazu eingesetzt werden, Licht in das Innere des Photobioreaktors einzukoppeln, als auch dazu, die phototrophen Organismen innerhalb des Photobioreaktors zu durchmischen oder abzuernten.

Gemäß einem ersten Aspekt der vorliegenden Erfindung wird eine Matte vorgeschlagen, welche eine Mehrzahl von ersten Fasern sowie eine Mehrzahl von zweiten Fasern aufweist. Die ersten Fasern sind entlang ihrer Längsrichtung lichtleitend und dazu ausgebildet, in Längsrichtung geleitetes Licht zumindest teilweise quer zur Längsrichtung seitlich auszukoppeln. Die zweiten Fasern sind entlang ihrer Längsrichtung elektrisch leitfähig.

Unter einer Matte kann dabei ein flächenartiges, dünnes Gebilde verstanden werden, bei dem mehrere Fasern miteinander mechanisch verbunden sind. Die Matte kann beispielsweise als Gewebe, Geflecht oder Gewirke ausgebildet sein. Die Matte kann mechanisch flexibel und insbesondere quer zu ihrer Erstreckungsfläche biegbar sein.

Unter einer Faser kann ein im Verhältnis zu seiner Länge dünnes und flexibles Gebilde verstanden werden. Fasern können aus verschiedensten Materialien bestehen. Aufgrund der geringen Dicke einer typischen Faser können dabei auch Materialien zum Einsatz kommen, die normalerweise bei größeren Massen hart oder spröde sind, ohne dass die Flexibilität der Faser beeinträchtigt würde. Insbesondere können Fasern aus Glas, Kohlenstoff, Kunststoffen oder Metallen bestehen. Auch aus Metall bestehende Drähte, Litzen, etc. sollen in diesem Zusammenhang als Fasern angesehen werden.

Die ersten Fasern sollen zumindest in ihrem Kern aus einem Material bestehen, welches für Licht, insbesondere Licht in einem Wellenbereich von 300 nm bis 1200 nm, insbesondere 400 mm bis 800 nm, weitgehend transparent ist. Orientiert am Einsatzzweck sollte die optische Transparenz dabei ausreichend hoch sein, so dass Licht von einem Ende der Faser durch die Faser hindurch bis an ihren Bestimmungsort ohne signifikante Absorptionsverluste, das heißt mit Absorptionsverlusten von beispielsweise weniger als 20%, geleitet werden kann. Insbesondere können die ersten Fasern als Glasfasern oder Polymerfasern aus einem transparenten Polymer ausgebildet sein.

Die zweiten Fasern können gleiche oder andere Durchmesser aufweisen wie die ersten Fasern und/oder können zumindest teilweise aus gleichen Materialien aufgebaut sein, wie die ersten Fasern. Die zweiten Fasern sollen jedoch zumindest ergänzend auch mit einem Material ausgebildet sein, welches eine elektrische Leitfähigkeit entlang der Längsrichtung der zweiten Fasern bewirkt.

Gemäß der Erfindung sind die zweiten Fasern als Kohlefasern ausgebildet, wie sie auch zur Herstellung von CFK-Geweben verwendet werden. Solche Kohlefasern sind einfach verarbeitbar und chemisch beständig. Insbesondere für eine Verwendung in einem Photobioreaktor sind Kohlefasern auch wegen ihrer Eignung zur Wasserzerlegung (Graphit) in Wasserstoff und Sauerstoff-Atome bzw. -moleküle geeignet. Außerdem bilden Kohlefasern im Allgemeinen keine Metallionen in einer Algen-Nährlösung, was bei einer Verwendung in einem Photobioreaktor ein entscheidender Vorteil bei einer Prozessoptimierung sein kann, da Metallionen giftig wirken können und allgemein negativ wirken und bei einer späteren Algenverwertung als Rohmasse hinderlich sein können.

Die zweiten Fasern werden mit einem elektrisch leitfähigen Polymer oder mit einem Edelmetall ausgebildet. Solche elektrisch leitfähigen Polymere sind im Allgemeinen gut verarbeitbar und chemisch beständig.

Beispielsweise können sie als Edelstahldraht ausgebildet sein. Derartige Metallfasern weisen im Allgemeinen eine hohe chemische Beständigkeit und eine starke optische Reflektivität auf, wodurch sie beispielsweise bei der Verwendung in einem Photobioreaktor als Spiegel an der Faserrückseite zum verbesserten Lichtauskoppeln an einer gegenüberliegenden gebogenen Lichtseite einer lichtleitenden Faser wirken können.

In einer weiteren Ausgestaltung können die zweiten Fasern in einem radial innen liegenden Bereich mit einem elektrisch isolierenden Material ausgebildet sein und in einem radial weiter außen liegenden Bereich mit einer elektrisch leitfähigen Schicht beschichtet sein. Mit anderen Worten braucht nicht die gesamte zweite Faser aus einem elektrisch leitfähigen Material zu bestehen, sondern es kann genügen, lediglich einen Teilbereich, beispielsweise eine Oberfläche der Faser, mit einem elektrisch leitfähigen Material zu beschichten. Zum Beispiel kann eine aus einem elektrisch isolierenden Material im Kern bestehende Faser mit einer dünnen Metallschicht beschichtet werden.

In einer weiteren Ausgestaltung können die zweiten Fasern derart ausgestaltet sein, dass sie in einem radial innen liegenden Bereich lichtleitend sind und in einem radial weiter außen liegenden Bereich mit einer elektrisch leitfähigen und optisch transparenten Schicht beschichtet sind. Derart ausgestaltete zweite Fasern können sowohl dazu verwendet werden, Licht zu leiten und an einem Bestimmungsort auszukoppeln, als auch elektrischen Strom zu führen bzw. elektrische Felder zu generieren. Beispielsweise kann eine zweite Faser in ihrem Kern als Glasfaser ausgebildet sein. An einer Oberfläche einer zweiten Faser kann eine optisch transparente Schicht beispielsweise aus Zinkoxid, Zinnoxid oder Indiumoxid ausgebildet sein, die eine ausreichend hohe elektrische Leitfähigkeit aufweist.

Die ersten und zweiten Fasern können miteinander zu einer Matte verwoben sein. Mit anderen Worten können die ersten und zweiten Fasern eine Gewebematte bilden. Die ersten Fasern können dabei als Kettfäden und die zweiten Fasern als Schussfäden, oder umgekehrt, verwoben sein. In einem Gewebe befinden sich die ersten und zweiten Fasern in einer regelmäßig geordneten Anordnung. Die ersten Fasern verlaufen dabei quer, im Regelfall näherungsweise senkrecht, zu den zweiten Fasern. In einem Gewebe sind die ersten und zweiten Fasern stabil miteinander verbunden, so dass eine solche Matte einfach gehandhabt und verarbeitet werden kann.

In der Matte können die zweiten Fasern vorzugsweise parallel zueinander angeordnet sein. Bei einer als Gewebe ausgestalteten Matte können die zweiten Fasern beispielsweise jeweils als Kettfäden oder jeweils als Schussfäden parallel zueinander verlaufen. Ein mit solchen parallelen zweiten Fasern erzeugtes elektrisches Feld kann besonders homogen bzw. linear sein.

Gemäß einer Ausgestaltung weist die Mehrzahl von zweiten Fasern wenigstens eine erste, eine zweite und eine dritte Untergruppe von zweiten Fasern auf. Die Untergruppen sind dabei untereinander elektrisch isoliert. Die Fasern jeder Untergruppe können vorzugsweise zu mehreren Bündeln zusammengefasst sein, wobei insbesondere jedes Bündel von Fasern einer Untergruppe von einem Bündel von Fasern einer anderen Untergruppe elektrisch isoliert angeordnet und/oder ausgebildet sein kann.

In einer solchen Ausgestaltung kann die Mehrzahl von zweiten Fasern der ersten, zweiten und dritten Untergruppe in einem zyklischen Muster angeordnet sein. Beispielsweise kann parallel zu Fasern oder einem Bündel von Fasern der ersten Untergruppe eine Faser bzw. ein Bündel von Fasern der zweiten Untergruppe angeordnet sein und parallel zu dieser wiederum eine Faser bzw. ein Bündel von Fasern der dritten Untergruppe, bevor dann wieder eine Faser bzw. ein Bündel von Fasern der ersten Untergruppe folgt, usw.

Gemäß einem zweiten Aspekt der vorliegenden Erfindung wird eine Mattenanordnung vorgeschlagen, welche eine Ausgestaltung der oben beschriebenen Matte sowie eine elektrische Spannungsquelle aufweist, welche mit den zweiten Fasern dieser Matte elektrisch verbunden ist.

Die Spannungsquelle kann dabei wenigstens drei Elektroden aufweisen, wobei die Mehrzahl von zweiten Fasern wiederum in eine erste, eine zweite und eine dritte Untergruppe von zweiten Fasern unterteilt sein kann und diese Untergruppen untereinander elektrisch isoliert sein können. In einer solchen Ausgestaltung kann jede der Untergruppen mit lediglich mit einer der Elektroden elektrisch verbunden sein und die Spannungsquelle beispielsweise dazu ausgelegt sein, in den mit den Elektroden verbundenen zweiten Fasern einen Drehstrom zu generieren. Unter einem Drehstrom kann dabei ein Mehrphasenwechselstrom verstanden werden, der aus mehreren, beispielsweise drei, einzelnen Wechselströmen oder Wechselspannungen gleicher Frequenz besteht, welche zueinander in ihren Phasenwinkeln fest, beispielsweise um 120°, verschoben sind. Ein derart in den zweiten Fasern generierter Drehstrom kann angrenzend an die Fasern zyklisch variierende elektrische Felder generieren. Insbesondere kann ein wanderndes elektrisches Wechselfeld erzeugt werden.

Gemäß einem weiteren Aspekt der vorliegenden Erfindung wird ein Photobioreaktor zur Kultivierung von phototrophen Organismen vorgeschlagen, der einen Behälter sowie eine der zuvor beschriebenen Mattenanordnungen aufweist. Der Behälter ist dabei dazu ausgebildet, die phototrophen Organismen zusammen mit einer Nährlösung aufzunehmen. Die Matten der Mattenanordnung sind innerhalb des Behälters angeordnet.

Bei einem solchen Photobioreaktor kann die Mattenanordnung einerseits dazu verwendet werden, Licht einer außen liegenden Lichtquelle durch die ersten Fasern der Matten ins Innere, das heißt in die in dem Behälter aufgenommene Nährlösung einzuspeisen und damit die phototrophen Organismen zu versorgen. Andererseits können über die elektrische Spannungsquelle der Mattenanordnung in den zweiten Fasern geeignete elektrische Spannungen bzw. Ströme erzeugt werden, um angrenzend an die Matten zeitlich variierende elektrische Felder zu generieren, mithilfe derer elektrisch geladene Bestandteile der Nährlösung oder elektrisch geladene phototrophe Organismen selbst bewegt werden können.

In einer Ausgestaltung kann die Spannungsquelle dazu ausgelegt sein, in den mit den Elektroden verbundenen zweiten Fasern einen Drehstrom derart zu generieren, dass angrenzend an die zugehörige Matte ein wanderndes elektrisches Wechselfeld erzeugt wird, dessen Wanderrichtung beispielsweise hin zu einem Erntebereich des Photobioreaktors gerichtet ist. Mit anderen Worten können die von der Spannungsquelle an die zweiten Fasern anzulegenden elektrischen Spannungen derart gesteuert werden, dass die von den zweiten elektrischen Fasern generierten elektrischen Felder zeitlich und räumlich derart variieren, dass sich in Summe ein wanderndes elektrisches Wechselfeld ergibt. In einem solchen wandernden elektrischen Wechselfeld bewegen sich Bereiche gleichen elektrischen Potenzials kontinuierlich und vorzugsweise mit gleichbleibender Wanderrichtung. Die Spannungsquelle kann dabei derart gesteuert werden, dass die Wanderrichtung hin zu einem Bereich des Photobioreaktors führt, aus dem gereifte phototrophe Organismen aus dem Photobioreaktor entnommen, das heißt geerntet, werden können.

Es wird darauf hingewiesen, dass mögliche Vorteile und Merkmale von Ausführungsformen der Erfindung hierin teilweise mit Bezug auf einen erfindungsgemäßen Photobioreaktor und teilweise mit Bezug auf eine erfindungsgemäße Matte bzw. Mattenanordnung beschrieben sind. Ein Fachmann wird erkennen, dass die verschiedenen Merkmale in geeigneter Weise kombiniert, übertragen beziehungsweise ausgetauscht werden können, um zu weiteren Ausführungsformen der Erfindung zu gelangen.

### KURZE BESCHREIBUNG DER ZEICHNUNGEN

Nachfolgend werden Ausführungsformen der Erfindung unter Bezugnahme auf die beigefügten Zeichnungen beschrieben, wobei weder die Beschreibung noch die Zeichnungen als die Erfindung einschränkend auszulegen sind.
Figur 1 zeigt einen Photobioreaktor gemäß einer Ausführungsform der vorliegenden Erfindung.
Figur 2 zeigt ausschnittsweise eine Mattenanordnung gemäß einer Ausführungsform der vorliegenden Erfindung
Figuren 3(a) bis (d) zeigen verschiedene Ausgestaltungen von Fasern für eine Matte gemäß einer Ausführungsform der vorliegenden Erfindung.
Figur 4 zeigt ein Photobioreaktorsystem gemäß einer Ausführungsform der vorliegenden Erfindung.

Die Figuren sind lediglich schematisch und nicht maßstabsgetreu. Gleiche Bezugszeichen bezeichnen in den unterschiedlichen Figuren gleiche beziehungsweise gleichwirkende Merkmale.

### BESCHREIBUNG BEVORZUGTER AUSFÜHRUNGSFORMEN

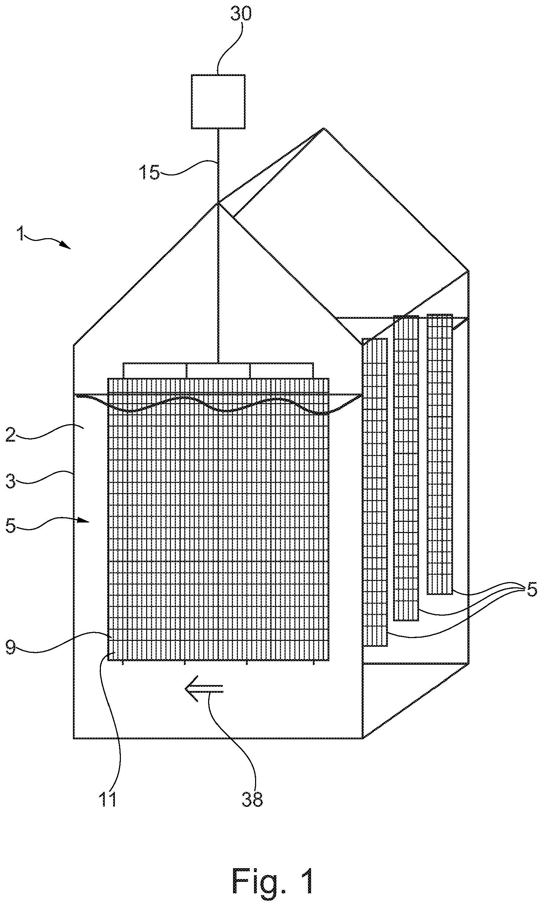

Figur 1 zeigt eine schematisierte perspektivische Ansicht eines Photobioreaktors 1 gemäß einer Ausführungsform der vorliegenden Erfindung. Der Photobioreaktor 1 weist einen Behälter 3 auf, in dem phototrophe Organismen zusammen mit einer Nährlösung 2 aufgenommen werden können. In dem Behälter 3 sind mehrere Matten 5 näherungsweise parallel zueinander und beabstandet voneinander angeordnet. Jede der Matten 5 ist mit einer Vielzahl von ersten lichtleitenden Fasern 9 ausgebildet, welche derart angeordnet und ausgebildet sind, dass Licht, welches beispielsweise über einen gemeinsamen aus dem Behälter 3 heraus geführten Lichtleiter 15 in Enden der Fasern 9 eingekoppelt wird, zumindest teilweise seitlich aus den Fasern 9 und damit quer zur Oberfläche der Matten 5 austritt. Jede Matte 5 weist ferner eine Vielzahl zweiter Fasern 11 auf, welche entlang ihrer Längsrichtung elektrisch leitfähig sind.

Der Behälter 3 kann eine beliebige Geometrie aufweisen. Beispielsweise kann der Behälter, wie in Figur 1 dargestellt, quaderförmig beziehungsweise kubusförmig ausgestaltet sein. Alternativ kann der Behälter 3 auch zylindrisch, kugelförmig oder mit einer anderen Form ausgebildet sein.

Der Behälter 3 kann dabei eine geeignete Geometrie aufweisen, bei der ein großes Volumen bei gleichzeitig verhältnismäßig kleiner Oberfläche in dem Behälter 3 aufgenommen werden kann. Insbesondere kann eine Tiefe des Behälters 3 größer sein als seitliche Abmessungen beziehungsweise die Grundfläche des Behälters 3. Die Tiefe des Behälters 3 soll dabei in einer Richtung quer zu einer Haupterstreckungsebene der Lichtleitermatte gemessen werden. Insbesondere kann der Behälter 3 in jeder Raumrichtung, das heißt in Höhe, Breite und Tiefe Abmessungen von mehr als 50 cm, vorzugsweise mehr als 1 m aufweisen.

Zumindest in einem unteren Bereich sollte der Behälter 3 dicht ausgeführt sein, so dass flüssige Nährlösung 2 zusammen mit den darin aufgenommenen phototrophen Organismen in dem Behälter 3 gehalten werden können. In einem oberen Bereich kann der Behälter 3, wie in Figur 1 dargestellt, ebenfalls geschlossen und dicht ausgeführt sein, so dass ein in sich geschlossener Photobioreaktor 1 gebildet wird. Alternativ kann der Behälter 3 jedoch auch nach oben hin offen sein, um einen offenen Photobioreaktor zu bilden. Wände des Photobioreaktors 1 (in Figur 1 zur besseren Veranschaulichung lediglich umrandet wiedergegeben, um eine Sicht auf innen liegende Komponenten des Photobioreaktors zu ermöglichen) können aus beliebigen fluiddichten Materialien wie beispielsweise Kunststoff oder Metall ausgebildet sein und brauchen nicht notwendigerweise lichtdurchlässig zu sein.

Jede der Matten 5 kann aus einer Vielzahl von lichtleitenden ersten Fasern 9 und elektrisch leitfähigen zweiten Fasern 11 zusammengesetzt sein. Die ersten Fasern 9 können dabei untereinander und mit den zweiten Fasern 11 auf unterschiedliche Weise fest oder lose miteinander verbunden sein. Die Matte 5 kann beispielsweise in Form eines Gewebes, eines Gewirkes, eines Vlies oder einer anderen 2- oder 3-dimensionalen Struktur, beispielsweise einer Wabenstruktur, bereitgestellt sein. Die Matte 5 ist dabei beispielsweise flächig ausgebildet, wobei eine Dicke quer zu der Haupterstreckungsrichtung der Fläche weniger als 10 mm, vorzugsweise weniger als 2 mm betragen kann. Die Matte ist in sich flexibel und biegsam und weist diesbezüglich ähnliche mechanische Eigenschaften auf, wie eine Folie. Allerdings ist die Matte 5 dadurch, dass sie aus einer Vielzahl von Fasern zusammengesetzt ist, fluiddurchlässig, das heißt, Fluid beispielsweise in Form der Nährlösung kann langsam durch die Matte 5 hindurch strömen.

Die einen Teil der Matte 5 bildenden ersten Fasern 9 sind zumindest in ihrem Inneren, das heißt, in einem Kern, gut lichtleitend, das heißt, sie weisen eine hohe optische Transparenz auf. Die Fasern können aus transparenten Materialien wie zum Beispiel Glas oder einem transparenten Kunststoff, insbesondere einem transparenten Polymer wie PMMA (Polymethylmethacrylat) bestehen. Die ersten Fasern 9 bzw. Kerne der ersten Fasern 9 können Durchmesser im Bereich von wenigen Mikrometern bis hin zu wenigen Millimetern aufweisen. Typische Durchmesser liegen im Bereich von 0,25 bis 2 mm, insbesondere 5 bis 30 µm. Jede der ersten Fasern 9 kann stark biegsam sein und beispielsweise in Krümmungsradien von weniger als 10 mm gekrümmt werden.

Um Licht im Inneren der ersten Faser 9 leiten zu können, kann die erste Faser 9 mit einer als "cladding" genannten Schicht umhüllt sein, welche einen niedrigeren optischen Brechungsindex aufweist als ein Material im Kern der erste Faser 9. In flachen Winkeln auf ein derartiges cladding auftreffendes Licht wird durch Totalreflexion wieder in den Kern der Faser zurückgeleitet und kann sich somit in einer länglichen Faser über weite Strecken hin ausbreiten.

Allerdings wird es für den speziellen Einsatz von Matten 5 in einem erfindungsgemäßen Photobioreaktor auch als möglich erachtet, lichtleitende Fasern ohne ein solches cladding vorzusehen, da angenommen wird, dass die die einzelnen Fasern umgebende Nährlösung ebenfalls einen geeigneten optischen Brechungsindex aufweisen dürfte, so dass es zur gewünschten Totalreflexion kommt.

Die lichtleitenden ersten Fasern 9 können mit einer möglichst glatten Oberfläche ausgebildet sein, um beispielsweise zu verhindern, dass sich Ablagerungen oder Schmutz an einzelnen Fasern anhaften können. Gegebenenfalls können die Fasern hydrophob beschichtet sein, zum Beispiel mit einer Schicht aus Titandioxid (TiO₂) überzogen sein. Auch eine Beschichtung mit einem eine Kratzfestigkeit erhöhenden Material kann vorgesehen sein. Etwaige Beschichtungen können beispielsweise mit Plasmaprozessen, einer Sol-Gel-Technik oder durch Lackieren aufgebracht werden.

Wie weiter unten anhand konkreter Ausführungsbeispiele detaillierter erklärt werden wird, sind die Matten 5 beziehungsweise die darin verwendeten lichtleitenden ersten Fasern 9 derart ausgestaltet, dass in den ersten Fasern 9 geleitetes Licht zumindest teilweise seitlich, das heißt quer zu einer Oberfläche der Lichtleitermatte 5, ausgekoppelt wird. Ein Anteil des seitlich austretenden Lichts soll dabei in Bezug auf eine Gesamtmenge des aus den Fasern 9 der Lichtleitermatte 5 austretenden Lichts erheblich sein, beispielsweise mindestens 10%, vorzugsweise aber mindestens 50%, eventuell sogar mindestens 90% betragen. Ein seitlich aus der Lichtleitermatte 5 austretender Lichtanteil kann dabei vorzugsweise homogen über die Matte 5 verteilt seitlich aus dieser austreten. Anders ausgedrückt, kann das in eine einzelne erste Faser 9 eingekoppelte Licht möglichst entlang der gesamten Länge der ersten Faser verteilt aus dieser seitlich austreten.

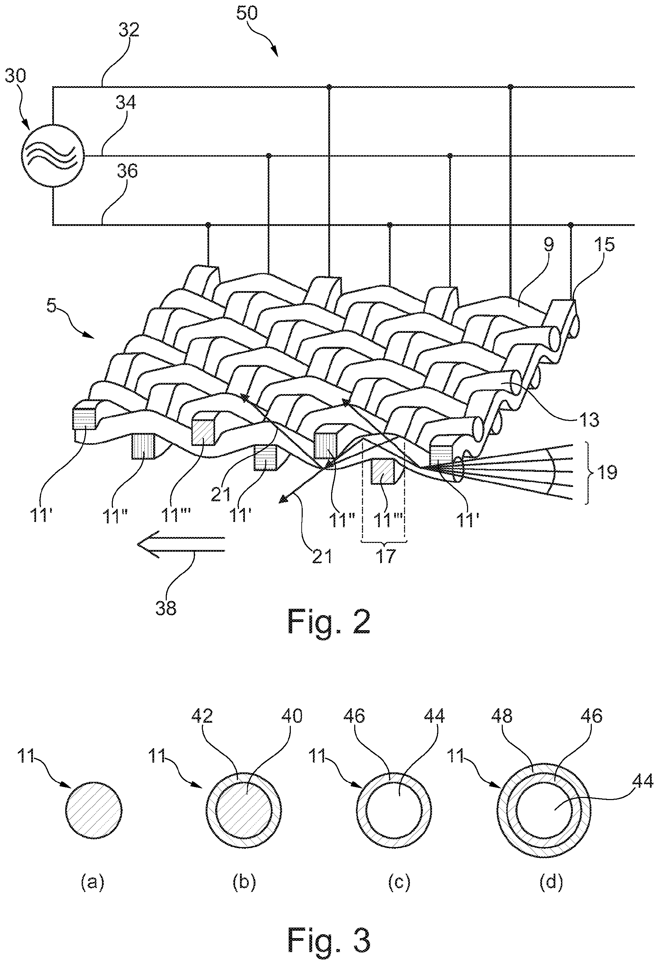

Figur 2 zeigt eine Ausgestaltung einer Mattenanordnung 50 mit einer Matte 5, bei der eine Vielzahl von lichtleitenden ersten Fasern 9 und elektrisch leitfähigen zweiten Fasern 11 miteinander zu einem Gewebe verwoben ist. Die Fasern 9, 11 des Gewebes können dabei in unterschiedlichen Webmustern miteinander verwoben sein. Dabei können die ersten Fasern 9 beispielsweise in Längsrichtung verlaufende Kettfäden 13 und die zweiten Faser 11 in Querrichtung verlaufende Schussfäden 15, oder umgekehrt, bilden.

Durch die verwobene Struktur werden die lichtleitenden ersten Fasern 9 dabei lokal derart gekrümmt, dass es zumindest in Bereichen 17 mit minimalem Krümmungsradius dazu kommt, dass Teile von in eine erste Faser 9 eingekoppeltem und in dieser in Längsrichtung der Faser geleitetem Licht 19 seitlich aus der Faser 9 ausgekoppelt werden. Die ausgekoppelten Lichtanteile 21 werden dabei quer zur Erstreckungsrichtung der Matte 5 abgestrahlt und können somit angrenzende Volumina innerhalb des Behälters 3 des Photobioreaktors 1 beleuchten.

Ein seitliches Auskoppeln von Licht aus einzelnen lichtleitenden Fasern 9 kann auch dadurch erreicht werden, dass in den lichtleitenden ersten Fasern 9 lokale Brechungsindexvariationen ausgebildet werden. Mit anderen Worten werden die ersten Fasern 9 derart hergestellt oder bearbeitet, dass Licht, welches sich im Inneren der ersten Fasern entlang deren Länge ausbreitet, Bereiche mit unterschiedlichen Brechungsindizes durchläuft beziehungsweise auf solche Bereiche trifft.

Die Brechungsindexvariationen können dabei lediglich an der Oberfläche einer Faser vorgesehen sein oder alternativ sich auch bis ins innere Volumen der Faser erstrecken.

Beispielsweise kann eine erste Faser an ihrer Außenoberfläche angeschliffen, geritzt, gekerbt oder ähnliches werden, so dass es im Bereich dieser Formänderungen der Fasern zu der gewünschten Brechungsindexvariation kommt. Dabei kann gegebenenfalls ein an einer Oberfläche der ersten Faser vorgesehenes cladding lokal entfernt werden, wodurch ein seitliches Auskoppeln von Lichtanteilen weiter begünstigt wird.

Alternativ kann eine Dichte der ersten Faser lokal beispielsweise durch temporäres lokales Erhitzen mittels eines Lasers verändert werden, was auch als Laser-Grating oder Fiber-Grating bezeichnet wird. Hierbei braucht eine außen liegende Oberfläche der Faser nicht modifiziert werden, insbesondere nicht geometrisch verändert werden und kann glatt bleiben, so dass keine Gefahr von lokalen Schmutzanlagerungen provoziert wird. Ähnliche Effekte können durch ein lokales Anschmelzen der Oberfläche einer Faser, insbesondere bei Polymerfasern, erreicht werden.

Eine weitere Möglichkeit zum lokal seitlichen Auskoppeln von Lichtanteilen kann durch Einbetten von mikroskopisch kleinen Streuzentren oder Fluoreszenzzentren in lichtleitende erste Fasern 9 implementiert werden. Streuzentren können dabei winzige Partikel aus vorzugsweise stark optisch reflektierendem Material, beispielsweise kleinste Metallpartikel sein. Fluoreszenzzentren können beispielsweise Partikel aus einem fluoreszierenden Material sein.

Wie in Figur 1 dargestellt, können innerhalb des Behälters 3 eines Photobioreaktors 1 mehrere Matten 5 gleichmäßig verteilt über ein gesamtes Volumen des Behälters 3 angeordnet werden. Die Matten 5 erstrecken sich dabei in näherungsweise parallelen Ebenen zueinander, beispielsweise parallel zu Ebenen von Seitenwänden des Behälters 3. Ein Abstand zwischen benachbarten Matten 5 kann dabei vorzugsweise geringer als 20 cm sein, so dass über weite Bereiche des Behälters 3 hin jeder Ort innerhalb des Behälters 3 höchstens 10 cm von einer der Matten 5 entfernt ist. Auf diese Weise kann vorzugsweise das gesamte Volumen der in dem Behälter 3 aufgenommenen Nährlösung oder zumindest große Anteile davon gleichmäßig mit Licht, welches durch den gemeinsamen Lichtleiter 11 in den Behälter 3 eingebracht und dann durch seitliche Auskopplung aus den Matten 5 in die Nährlösung eingestrahlt wurde, versorgt werden.

Zusätzlich zu den lichtleitenden ersten Fasern 9 weist die Matte 5 elektrisch leitfähige zweite Fasern 11 auf. Bei dem in Figur 2 beispielhaft dargestellten Gewebe der Matte 5 verlaufen die elektrisch leitfähigen zweiten Fasern 11 quer, insbesondere im Wesentlichen rechtwinklig zu den ersten Fasern 9. Die zweiten Fasern 11 verlaufen dabei im Wesentlichen parallel zueinander.

Im dargestellten Beispiel sind die zweiten Fasern 11 dabei in eine erste Untergruppe 11', eine zweite Untergruppe 11" und eine dritte Untergruppe 11‴ unterteilt. Die zweiten Fasern 11', 11", 11‴ der ersten, zweiten und dritten Untergruppe sind dabei in einem zyklischen Muster angeordnet. Bei dem in Figur 2 dargestellten Beispiel mit drei Untergruppen ist benachbart zu einer zweiten Faser 11' der ersten Untergruppe eine Faser 11" der zweiten Untergruppe und dann einer Faser 11‴ der dritten Untergruppe angeordnet, bevor sich der Zyklus wiederholt und wieder eine Faser 11' der ersten Gruppe folgt.

Die Mattenanordnung 50 weist zusätzlich zu der Matte 5 eine elektrische Spannungsquelle 30 auf. Die Spannungsquelle 30 ist derart ausgestaltet, dass an drei verschiedenen Elektroden 32, 34, 36 elektrische Wechselspannungen anliegen, die jeweils um 120° phasenverschoben zueinander sind. Jede der zweiten Fasern 11', 11", 11‴ einer der drei Untergruppen ist jeweils mit einer der drei Elektroden 32, 34, 36 elektrisch verbunden und gegenüber zweiten Fasern anderer Untergruppen elektrisch isoliert. Durch die an den zweiten Fasern 11', 11", 11‴ anliegenden Wechselspannungen werden in der Nähe dieser zweiten Fasern zeitlich variierende elektrische Felder erzeugt, wobei zeitlich und räumlich variierende Feldgradienten auftreten.

Dadurch, dass in der Matte 5 nicht nur zwei, sondern wenigstens drei verschiedene Untergruppen von zweiten Fasern 11', 11", 11‴ vorgesehen sind und diese dementsprechend mit wenigstens drei Elektroden 32, 34, 36 mit daran anliegenden phasenverschobenen Wechselspannungen verschaltet sind, können räumlich fortlaufende, sozusagen ineinander gereihte elektrische Feldstrukturen in Form eines wandernden elektrischen Wechselfeldes, teilweise auch als "travelling wave" bezeichnet, erzeugt werden. Der von der Spannungsquelle 30 erzeugte Drehstrom bzw. die Drehspannung kann dabei ähnlich wie in einem beispielsweise dreiphasigen Elektromotor für ein wanderndes elektrisches Wechselfeld sorgen.

Ein solches wanderndes elektrisches Wechselfeld kann auf geladene Teilchen, welche sich in der Nähe der Matte 5 befinden, wirken und auf diese eine Kraft ausüben, um diese somit in eine Wanderrichtung 38 zu bewegen. Die Wanderrichtung 38 verläuft dabei im Allgemeinen rechtwinklig zur Längserstreckungsrichtung der zweiten Fasern 11.

Bei dem in Figur 1 dargestellten Beispiel verlaufen die zweiten Fasern 11 vertikal, so dass sich eine Wanderrichtung 38 in etwa parallel zum Boden des Behälters 3 des Photobioreaktors 1 ergibt. Phototrophe Organismen, welche häufig im Grundzustand oder zumindest in einem durch Licht angeregten Zustand ionisiert und somit elektrisch geladen sind, können aufgrund des von der Matte 5 generierten elektrischen Wechselfeldes entlang der Wanderrichtung 38 transportiert oder gefördert werden. Im dargestellten Beispiel können die phototrophen Organismen somit von einem Bereich rechts in dem Behälter 3, in dem diese Organismen beispielsweise anfänglich eingespeist werden, sukzessive hin zu einem Bereich links in dem Behälter 3 bewegt werden. Während dieses Bewegungsvorgangs werden die phototrophen Organismen kontinuierlich durch aus den ersten Fasern 9 der Matte 1 austretendes Licht versorgt und können somit sukzessive reifen. Ein Bereich links in dem Behälter 3 kann als Erntebereich ausgestaltet sein (in Figur 1 nicht spezifisch dargestellt), aus dem gereifte Organismen entnommen und einer weiteren Verarbeitung zugeführt werden können.

In einer alternativen Ausführungsform können die zweiten Fasern 11 horizontal angeordnet sein. In diesem Fall ergibt sich eine Wanderrichtung 38 in vertikaler Richtung, so dass elektrisch geladene Teilchen wie beispielsweise die phototrophen Organismen sukzessive entweder hin zu einer obenliegenden Oberfläche der Nährlösung 2 gefördert und dort z.B. abgefischt werden können oder sukzessive zu einem Boden des Behälters 3 gefördert werden können und dort z.B. abgesaugt werden können.

In den Figuren 3(a)-(d) sind verschiedene mögliche Ausgestaltungen von elektrisch leitfähigen zweiten Fasern 11 dargestellt.

Bei dem in Figur 3(a) dargestellten Beispiel besteht die gesamte Faser 11 aus einem elektrisch leitfähigen Material wie zum Beispiel Kohlenstoff bzw. Kohlenstoffverbindungen, einem elektrisch leitfähigen Polymer (z.B. Polyanilin) oder einem Metall.

Bei dem in Figur 3(b) dargestellten Beispiel ist ein innerer Bereich 40 der Faser 11 aus einem elektrisch leitfähigen Material aufgebaut und von einer elektrisch isolierenden Schicht 42, beispielsweise aus einem Dielektrikum bestehend, ummantelt. Die elektrisch isolierende Schicht 42 kann dabei verhindern, dass der unter einer elektrischen Spannung stehende innere Bereich 40 direkt in Kontakt beispielsweise mit einer flüssigen Nährlösung in einem Photobioreaktor kommt. Während ein erzeugtes elektrisches Feld durch die isolierende Schicht 42 hindurchtreten kann, kann durch das Vermeiden eines direkten elektrischen Kontakts zwischen dem inneren Bereich 40 und der Nährlösung vermieden werden, dass elektrische Ströme fließen und Elektrolyseprozesse einsetzen, welche die Nährlösung schädigen können. Ergänzend kann beispielsweise auch vermieden werden, dass aus einem den inneren Bereich 40 bildenden metallischen Kern z.B. Metallionen in die Nährlösung gelangen und diese schädigen.

Bei dem in Figur 3(c) dargestellten Beispiel ist ein radial innen liegender Bereich 44 mit einem elektrisch isolierenden Material ausgebildet. Beispielsweise kann dieser innen liegende Bereich 44 lichtleitend ausgebildet sein, zum Beispiel als Glasfaser. Der innen liegende Bereich 44 ist von einem außen liegenden Bereich 46 aus einem elektrisch leitfähigen Material, wie zum Beispiel einer Metallschicht, einer Kohlenstoffschicht oder einer Schicht aus elektrisch leitfähigem Polymer, umgeben. Falls der radial innen liegende Bereich 44 lichtleitend ausgestaltet ist, kann es vorteilhaft sein, den elektrisch leitfähigen umgebenden Bereich 46 mit einem transparenten Material wie zum Beispiel Zinnoxid, Zinkoxid oder Indiumoxid auszubilden, so dass die gleiche Faser sowohl zum Leiten von Licht im inneren Bereich 44 als auch zum Leiten von elektrischem Strom im äußeren Bereich 46 dienen kann. Der umgebende leitfähige Bereich kann auch vorteilhaft aus einem transparenten elektrisch leitendem Polymer bestehen wie z.B. Poly-3,4-Ethylendioxithiophen (PEDT) zur verbesserten Anpassung der Brechungsindizes.

Bei dem in Figur 3(d) dargestellten Beispiel ist der in Figur 3(c) dargestellte Aufbau ergänzend durch eine außen liegende Umhüllung aus einer elektrisch isolierenden Schicht 48 geschützt und gegenüber einem umgebenden Medium wie zum Beispiel einer Nährlösung elektrisch isoliert. Die elektrisch isolierende Schicht 48 kann dabei gegebenenfalls auch optisch transparent ausgebildet sein.

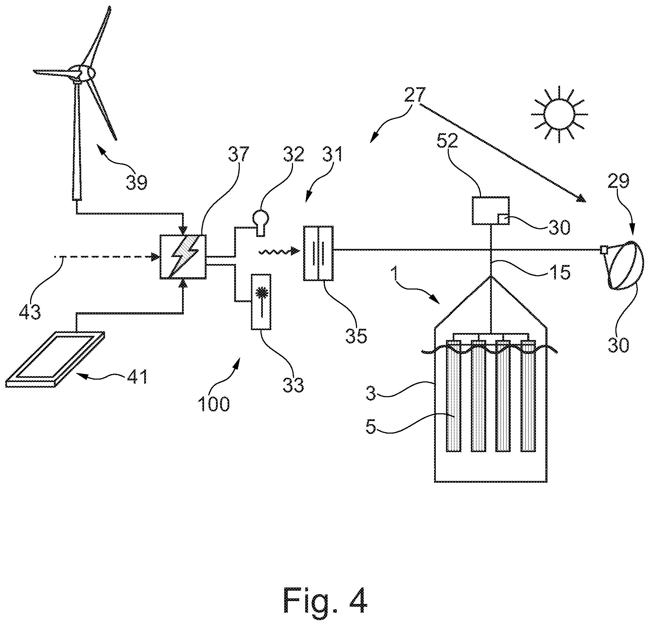

Figur 4 veranschaulicht schematisch ein Photobioreaktorsystem 100 gemäß einer Ausführungsform der vorliegenden Erfindung. Das Photobioreaktorsystem 100 weist einen erfindungsgemäßen Photobioreaktor 1 sowie eine Lichtquelle 27 auf. Die Lichtquelle 27 kann dabei ein oder mehrere Komponenten zum künstlichen Erzeugen von Licht oder zum Einsammeln natürlich erzeugten Lichts und anschließendem Einkoppeln dieses Lichts in einen gemeinsamen Lichtleiter 15 zum Versorgen des Photobioreaktors 1 aufweisen.

Einerseits kann die Lichtquelle 27 als eine Lichtquelle 29 zum Sammeln und Einkoppeln von Sonnenlicht in die lichtleitenden Fasern des Photobioreaktors 1 ausgestaltet sein. Eine solche Lichtquelle 29 kann beispielsweise als Sonnenkollektor 30 mit einem Hohlspiegel, der Sonnenlicht auf einen Empfänger fokussiert, ausgebildet sein. Zusätzlich oder alternativ können Lichtleitermatten zur Absorption des Sonnenlichtes in diesem Sinne als Lichtquelle gelten. Der Empfänger kann hierbei mit dem Lichtleiter 11 verbunden sein. Auf diese Weise kann bei Sonnenschein natürliches Licht einfach und energiesparend zur Beleuchtung des inneren Volumens des Photobioreaktors 1 genutzt werden.

Alternativ oder ergänzend hierzu kann die Lichtquelle 27 als Lichtquelle 31 zum künstlichen Erzeugen und Einkoppeln von Licht in lichtleitende erste Fasern 9 des Photobioreaktors 1 ausgestaltet sein. Eine solche künstliche Lichtquelle kann beispielsweise als LED 32 oder als Laser 33 ausgestaltet sein, welche Licht auf eine Anordnung 35 aus einem Polarisator und einem Abschatter einstrahlt, welche wiederum mit dem Lichtleiter 15 hin zu dem Photobioreaktor 1 verbunden ist.

Die künstlichen Lichtquellen 32, 33 können durch elektrischen Strom aus alternativen Quellen wie zum Beispiel durch Windkraft 39 oder durch Solarzellen 41 oder alternativ durch konventionellen Strom 43 versorgt werden. Der elektrische Strom kann dabei beispielsweise über eine Pufferbatterie 37 zwischengespeichert werden, so dass die künstliche Lichtquelle 31 den Photobioreaktor 1 auch bei mangelndem Sonnenschein belichten kann.

In dem Photobioreaktorsystem 100 ist ferner eine Steuerungseinheit 52 vorgesehen. Diese Steuerungseinheit 52 ist über den Lichtleiter 15 mit lichtleitenden Fasern 9 der Matten 5 in dem Photobioreaktor 1 verbunden und dazu ausgestaltet, Licht gezielt einzuspeisen. Außerdem beinhaltet die Steuerungseinheit 52 auch die Spannungsquelle 30, mit Hilfe der in den Matten 5 geeignete elektrische Wechselfelder erzeugt werden, um die mit phototrophen Organismen versetzte Nährlösung 2 permanent umzuwälzen und/oder die phototrophen Organismen gegebenenfalls für ein Abernten geeignet zu transportieren.

Ausführungsformen der vorliegenden Erfindung können unter anderem folgende Vorteile ermöglichen:
Ein Transport von phototrophen Organismen wie zum Beispiel Algen kann in einem flüssigen Nährmedium ohne externe Antriebe, Rührer oder dergleichen bewirkt werden. Es kann genügen, an den ohnehin für eine Lichteinspeisung vorgesehenen Matten 5 mithilfe der darin vorgesehenen zweiten elektrisch leitfähigen Fasern 11 wandernde elektrische Wechselfelder zu erzeugen, um die phototrophen Organismen zu bewegen. Es werden daher keine mechanisch bewegten Teile zum Durchmischen oder Transport benötigt. Dies kann unter anderem einen sehr viel kompakteren Aufbau für den Photobioreaktor ermöglichen. Beispielsweise lässt sich ein Abstand zwischen benachbarten Matten 5 sehr viel enger einstellen, als dies bei herkömmlichen Photobioreaktoren der Fall war, bei denen die mit phototrophen Organismen versetzte Nährlösung beispielsweise mithilfe eines Rührers umgerührt werden musste, da eine Fluiddynamik durch den intrinsischen Antrieb wesentlich verbessert werden kann.

Da die mit phototrophen Organismen versetzte Lösung innerhalb des Photobioreaktors in ständiger Bewegung gehalten werden kann, insbesondere nahe einer Oberfläche der Matten 5, kann eine Anhaftung an der Oberfläche der Matten 5 sehr gut unterbunden werden, was einer langen Anlagenlebensdauer zugutekommen kann, Wartungsintervalle minimieren kann und die Photonenströme innerhalb des Photobioreaktors aufrechterhalten kann.

Da kleinere Abstände zwischen den einzelnen licht-emittierenden Matten möglich sind und hierdurch beispielsweise ein Wirkungsgrad bei der Algenproduktion erhöht werden kann, kann insbesondere eine höhere Photoneneffizienz erreicht werden. Gleichzeitig kann gegebenenfalls mit niedrigeren Photonendichten gearbeitet werden, was wiederum eine Anlagenausbeute erhöhen kann. Ein Transport von phototrophen Organismen in einem beschriebenen Photobioreaktor kann mit nach außen hin elektrisch isolierten, aber im Innern elektrisch leitfähigen zweiten Fasern oder alternativ auch mit nach außen nicht-isolierten zweiten Fasern ausgeführt werden. Die nach außen isolierte Ausführung kann den Vorteil haben, dass sehr viel weniger elektrische Ladungen abfließen können und ein Betrieb des Transports der phototrophen Organismen energetisch sehr begünstigt werden kann.

Neben einem Transport der phototrophen Organismen kann auch ein integriertes Ernten ("harvesting") in besonderer Weise unterstützt werden.

## Patentansprüche

1. Matte (5), aufweisend:
eine Mehrzahl von ersten Fasern (9), welche entlang ihrer Längsrichtung lichtleitend sind und dazu ausgebildet sind, in Längsrichtung geleitetes Licht zumindest teilweise quer zur Längsrichtung seitlich auszukoppeln;
eine Mehrzahl von zweiten Fasern (11), welche entlang ihrer Längsrichtung elektrisch leitfähig sind und welche als Kohlefasern, mit einem elektrisch leitfähigen Polymer oder mit einem Edelmetall ausgebildet sind.

2. Matte nach Anspruch 1, wobei die ersten und zweiten Fasern (9, 11) miteinander verwebt sind.

3. Matte nach Anspruch 1 oder 2, wobei die zweiten Fasern (11) in einem radial innenliegenden Bereich (40) mit einem elektrisch isolierenden Material ausgebildet sind und in einem radial weiter außen liegenden Bereich (42) mit einer elektrisch leitfähigen Schicht beschichtet sind.

4. Matte nach einem der Ansprüche 1 bis 3, wobei die zweiten Fasern (11) in einem radial innenliegenden Bereich (44) lichtleitend sind und in einem radial weiter außen liegenden Bereich (46) mit einer elektrisch leitfähigen und optisch transparenten Schicht beschichtet sind.

5. Matte nach einem der Ansprüche 1 bis 4, wobei die zweiten Fasern (11) mit einer elektrisch isolierenden Schicht (48) umhüllt sind.

6. Matte nach einem der Ansprüche 1 bis 5, wobei die zweiten Fasern (11) parallel zueinander angeordnet sind.

7. Matte nach einem der Ansprüche 1 bis 6, wobei die Mehrzahl von zweiten Fasern (11) wenigstens eine erste, eine zweite und eine dritte Untergruppe von zweiten Fasern (1 1', 11", 11‴) aufweist, wobei die Untergruppen untereinander elektrisch isoliert sind.

8. Matte nach Anspruch 7, wobei die Mehrzahl von zweiten Fasern (11', 11", 11‴) der ersten, zweiten und dritten Untergruppe in einem zyklischen Muster angeordnet sind.

9. Mattenanordnung (50) aufweisend:
eine Matte (5) gemäß einem der Ansprüche 1 bis 8,
eine elektrische Spannungsquelle (30), welche mit den zweiten Fasern (11) elektrisch verbunden ist.

10. Mattenanordnung (50) nach Anspruch 9, wobei die Spannungsquelle (30) wenigstens drei Elektroden (32, 34, 36) aufweist,
wobei die Mehrzahl von zweiten Fasern (11) wenigstens eine erste, eine zweite und eine dritte Untergruppe von zweiten Fasern (11', 11 ` `, 11‴) aufweist, wobei die Untergruppen untereinander elektrisch isoliert sind,
wobei jede der Untergruppen mit lediglich mit einer der Elektroden (32, 34, 36) elektrisch verbunden ist, und
wobei die Spannungsquelle (30) dazu ausgelegt ist, in den mit den Elektroden (32, 34, 36) verbundenen zweiten Fasern (11', 11", 11‴) einen Drehstrom zu generieren.

11. Photobioreaktor (1) zur Kultivierung von phototrophen Organismen, wobei der Photobioreaktor (1) aufweist:
einen Behälter (3), in dem die phototrophen Organismen zusammen mit einer Nährlösung (2) aufgenommen werden können,
wenigstens eine Mattenanordnung (50) gemäß einem der Ansprüche 9 und 10, wobei Matten (5) der Mattenanordnung (50) innerhalb des Behälters (3) angeordnet sind.

12. Photobioreaktor (1) nach Anspruch 11, wobei die Spannungsquelle (30) dazu ausgelegt, in den mit den Elektroden (32, 34, 36) verbundenen zweiten Fasern einen (11', 11", 11‴) Drehstrom derart zu generieren, dass angrenzend an die zugehörige Matte (5) ein wanderndes elektrisches Wechselfeld erzeugt wird.

13. Photobioreaktor (1) nach Anspruch 12, wobei eine durch das wandernde elektrische Wechselfeld erzeugte Wanderrichtung (38) hin zu einem Erntebereich des Photobioreaktors (1) gerichtet ist.

## Claims

1. Mat (5), having:
a multiplicity of first fibres (9) which are light-conducting along their longitudinal direction and are designed to laterally outcouple light, conducted in the longitudinal direction, at least partially transversely to the longitudinal direction;
a multiplicity of second fibres (11) which are electrically conductive along their longitudinal direction and which are formed as carbon fibres, with an electrically conductive polymer or with a noble metal.

2. Mat according to Claim 1, wherein the first and second fibres (9, 11) are interwoven with each other.

3. Mat according to Claim 1 or 2, wherein the second fibres (11) in a radially inner region (40) are formed with an electrically insulating material and in a region (42) radially further to the outside are coated with an electrically conductive layer.

4. Mat according to Claim 1 or 3, wherein the second fibres (11) in a radially inner region (44) are light-conducting and in a region (46) radially further to the outside are coated with an electrically conductive and optically transparent layer.

5. Mat according to any of Claims 1 to 4, wherein the second fibres (11) are enveloped with an electrically insulating layer (48).

6. Mat according to any of Claims 1 to 5, wherein the second fibres (11) are arranged parallel to one another.

7. Mat according to any of Claims 1 to 6, wherein the multiplicity of second fibres (11) includes at least a first, a second and a third subgroup of second fibres (11', 11", 11‴), wherein the subgroups are electrically insulated from one another.

8. Mat according to Claim 7, wherein the multiplicity of second fibres (11', 11", 11‴) of the first, second and third subgroups are arranged in a cyclical pattern.

9. Mat arrangement (50), having:
a mat (5) according to any of Claims 1 to 8,
an electric voltage source (30) which is electrically connected to the second fibres (11).

10. Mat arrangement (50) according to Claim 9, wherein the voltage source (30) has at least three electrodes (32, 34, 36),
wherein the multiplicity of second fibres (11) includes at least a first, a second and a third subgroup of second fibres (11', 11", 11‴), wherein the subgroups are electrically insulated from one another,
wherein each of the subgroups is electrically connected to just one of the electrodes (32, 34, 36), and
wherein the voltage source (30) is designed to generate a three-phase current in the second fibres (11', 11'', 11‴) connected to the electrodes (32, 34, 36).

11. Photobioreactor (1) for culturing phototrophic organisms, wherein the photobioreactor (1) has:
a vessel (3) in which the phototrophic organisms can be accommodated together with a nutrient solution (2),
at least one mat arrangement (50) according to either of Claims 9 and 10, wherein mats (5) of the mat arrangement (50) are arranged within the vessel (3).

12. Photobioreactor (1) according to Claim 11, wherein the voltage source (30) is designed to generate a three-phase current in the second fibres (11', 11", 11‴) connected to the electrodes (32, 34, 36) in such a way that a travelling alternating electric field is produced adjacent to the associated mat (5).

13. Photobioreactor (1) according to Claim 12, wherein a travel direction (38) produced by the travelling alternating electric field is directed towards a harvesting region of the photobioreactor (1).

## Revendications

1. Natte (5), présentant :
une pluralité de premières fibres (9) qui sont conductrices de lumière le long de leur direction longitudinale et réalisées pour découpler latéralement la lumière guidée dans la direction longitudinale au moins en partie transversalement à la direction longitudinale ;
une pluralité de deuxièmes fibres (11) qui sont électriquement conductrices le long de leur direction longitudinale et qui sont réalisées sous forme de fibres de carbone avec un polymère électriquement conducteur ou avec un métal précieux.

2. Natte selon la revendication 1, dans laquelle les premières et les deuxièmes fibres (9, 11) sont tissées ensemble.

3. Natte selon la revendication 1 ou 2, dans laquelle les deuxièmes fibres (11) sont réalisées dans une zone (40) située radialement à l'intérieur avec un matériau électriquement isolant et sont recouvertes d'une couche électriquement conductrice dans une zone (42) située radialement plus à l'extérieur.

4. Natte selon l'une quelconque des revendications 1 à 3, dans laquelle les deuxièmes fibres (11) sont conductrices de lumière dans une zone (44) située radialement à l'intérieur et sont recouvertes d'une couche optiquement transparente dans une zone (46) située radialement plus à l'extérieur.

5. Natte selon l'une quelconque des revendications 1 à 4, dans laquelle les deuxièmes fibres (11) sont enveloppées d'une couche (48) électriquement isolante.

6. Natte selon l'une quelconque des revendications 1 à 5, dans laquelle les deuxièmes fibres (11) sont disposées en parallèle les unes aux autres.

7. Natte selon l'une quelconque des revendications 1 à 6, dans laquelle la pluralité de deuxièmes fibres (11) présente au moins un premier, un deuxième et un troisième sous-groupe de deuxièmes fibres (11', 11", 11"'), les sous-groupes étant électriquement isolés les uns par rapport aux autres.

8. Natte selon la revendication 7, dans laquelle la pluralité de deuxièmes fibres (11', 11", 11"') du premier, du deuxième et du troisième sous-groupe sont disposées selon un motif cyclique.

9. Agencement de nattes (50), présentant :
une natte (5) selon l'une quelconque des revendications 1 à 8,
une source de tension électrique (30) qui est reliée électriquement aux deuxièmes fibres (11).

10. Agencement de nattes (50) selon la revendication 9,
dans lequel la source de tension (30) présente au moins trois électrodes (32, 34, 36),
dans lequel la pluralité de deuxièmes fibres (11) présente au moins un premier, un deuxième et un troisième sous-groupe de deuxièmes fibres (11', 11", 11"'), les sous-groupes étant électriquement isolés les uns par rapport aux autres,
dans lequel chacun des sous-groupes est électriquement relié uniquement à l'une des électrodes (32, 34, 36), et
dans lequel la source de tension (30) est conçue pour produire un courant triphasé dans les deuxièmes fibres (11', 11", 11"') reliées aux électrodes (32, 34, 36) .

11. Photobioréacteur (1) permettant de cultiver des organismes phototrophes, le photobioréacteur (1) présentant :
un récipient (3) qui peut recevoir les organismes phototrophes conjointement avec un bouillon de culture (2),
au moins un agencement de nattes (50) selon l'une quelconque des revendications 9 et 10, dans lequel des nattes (5) de l'agencement de nattes (50) sont disposées à l'intérieur du récipient (3).

12. Photobioréacteur (1) selon la revendication 11, dans lequel la source de tension (30) est conçue pour produire un courant triphasé dans les deuxièmes fibres (11', 11", 11"') reliées aux électrodes (32, 34, 36) de telle sorte qu'un champ électrique alternatif progressif est produit à proximité de la natte (5) associée.

13. Photobioréacteur (1) selon la revendication 12, dans lequel une direction de progression (38) produite par le champ électrique alternatif progressif est tournée vers une zone de récolte du photobioréacteur (1).
